# EUROPEAN PATENT APPLICATION

(11) **EP 4 748 317 A1**
(43) Date of publication of application: **27.05.2026**
(21) Application number: 24214616.5
(22) Date of filing: 21.11.2024
(51) Int. Cl.: A61B 8/08, A61B 8/00

(54) **SYSTEM AND METHOD FOR DEFINING IMAGING REGION**

(71) Applicant: Koninklijke Philips N.V., 5656 AE Eindhoven (NL)
(72) Inventor: WEBER, Frank Michael, Eindhoven (NL); WAECHTER-STEHLE, Irina, Eindhoven (NL); WILD, Sebastian, 5656AG Eindhoven (NL); PETERS, Jochen, Eindhoven (NL); GOOSSEN, André, Eindhoven (NL); WEHLE, Simon, Eindhoven (NL); GESSERT, Nils Thorben, Eindhoven (NL); GROTH, Alexandra, Eindhoven (NL)
(74) Representative: Philips Intellectual Property & Standards

(57) **Abstract**

A system and method for defining at least one imaging region for assessing residual regurgitation. Medical imaging data of a heart having an implant device is obtained and processed to identify at least a position of the implant device. One or more locations of expected residual regurgitation are determined based at least one the identified position of the implant device and are used to define at least one imaging region for assessing residual regurgitation.

## Description

### FIELD OF THE INVENTION

The invention relates to the field of ultrasound imaging.

### BACKGROUND OF THE INVENTION

Mitral regurgitation and tricuspid regurgitation are types of heart valve disease in which the mitral valve or tricuspid valve respectively does not fully close, allowing blood to flow backwards through the respective valve. Mitral regurgitation and tricuspid regurgitation can be treated by fitting a device in or around the relevant valve. For instance, tricuspid regurgitation may be treated by placing a clip such that the opposing leaflets of the tricuspid valve are pulled together by the clip.

Once a device has been fitted in or around a damaged valve, residual regurgitation may be assessed to determine whether the valve has been sufficiently repaired. If the residual regurgitation is too high, a second device (e.g. a second clip) may be fitted to further reduce regurgitation.

Residual regurgitation is difficult to assess. 3D color Doppler over a large enough region to reliably capture the location of regurgitation does not typically yield a high enough frame rate to reliably capture the time of the peak of the regurgitant jet; reducing the size of the image region to achieve a high enough frame rate increases a likelihood that the image region does not capture the correct location.

Therefore, clinicians generally use 2D color or X-plane color imaging to assess residual regurgitation. 2D color and X-plane color imaging enables a large scanning area in the respective scanning planes to be imaged at a high frame rate; however, neither 2D color imaging nor X-plane imaging covers the entire 3D volume in which regurgitation may occur, so the images acquired may miss the location of the peak of the regurgitant jet. Further, sweeping 2D scans over the area using visual assessment is error-prone, as the information is difficult to compare.

There is therefore a need for improved imaging of residual regurgitation.

### SUMMARY OF THE INVENTION

The invention is defined by the claims.

According to examples in accordance with an aspect of the invention, there is provided a processing system for defining at least one imaging region for assessing residual regurgitation, the processing system being configured to: obtain medical imaging data of a heart of a subject, the medical imaging data capturing an implant device implanted in the heart; process the medical imaging data to identify a position of the implant device; determine one or more locations of expected residual regurgitation based on at least the identified position of the implant device; and define, based on the one or more locations of expected residual regurgitation, at least one imaging region for assessing residual regurgitation.

The inventors have recognized that the position of an implant device implanted in a heart for valve repair provides a good indication of suitable measurement locations for assessing residual regurgitation. The implant device may be any suitable valve repair device, such as a clipping device.

Defining an imaging region based on one or more locations of expected residual regurgitation enables a size of the imaging region to be small enough (in the case of Doppler imaging) to acquire imaging data at a high frame rate (i.e. high enough that there is a high probability of capturing the time of the peak of the regurgitant jet) and the position of the imaging region to have a high likelihood of capturing the spatial location of the peak of the regurgitant jet.

The medical imaging data used to identify the position of the implant device may be any medical imaging data from which the position of objects captured by the medical imaging data may be reconstructed in 3D space. For instance, the medical imaging data may be 3D medical imaging data or 2D imaging data from which 3D position information may be reconstructed (e.g. fluoroscopy imaging data).

In some examples, the processing system is further configured to process the medical imaging data to identify an orientation of the implant device; and the determination of the one or more locations of expected residual regurgitation is further based on the identified orientation of the implant device.

In some examples, the processing system is configured to identify the position of the implant device by processing the medical imaging data using a first segmentation algorithm.

The first segmentation algorithm may, for example, be a machine-learning algorithm trained to segment implant devices in ultrasound images.

In some examples, the medical imaging data is 3D ultrasound medical imaging data.

In some examples, the processing system is further configured to process the medical imaging data to identify a position of one or more anatomical features; and the determination of the one or more locations of expected residual regurgitation is further based on the identified position of the one or more anatomical features.

The use of anatomical information in addition to the position (and, optionally, orientation) of the implant device may further improve the determination of one or more locations at which residual regurgitation is most likely to occur.

In some examples, the processing system is configured to identify the position of the one or more anatomical features by processing the medical imaging data using a second segmentation algorithm.

The second segmentation algorithm may, for example, be a machine-learning algorithm trained to segment anatomical features in ultrasound images. In some examples, where a first segmentation algorithm is used to identify the position of the implant device, the first segmentation algorithm and the second segmentation algorithm may be the same segmentation algorithm (i.e. a single segmentation algorithm may be used both to identify the position of the implant device and to identify the position of the one or more anatomical features). Alternatively, separate segmentation algorithms may be used to identify the position of the implant device and the one or more anatomical features, or the position of the implant device may be determined using a different technique.

In some examples, the processing system is further configured to obtain pathology information for the subject; and the determination of the one or more locations of expected residual regurgitation is further based on the pathology information.

The use of pathology information in addition to the position of the implant device (and, optionally, orientation of the implant device and/or the position of one or more anatomical features) may further improve the determination of one or more locations at which residual regurgitation is most likely to occur.

In examples in which a position of one or more anatomical features is determined, the processing system may be configured to obtain the pathology information based on the identified position of the one or more anatomical features. Alternatively, the pathology information may be obtained from an electronic medical record for the subject or via user input.

In some examples, the processing system is configured to define the at least one imaging region for assessing residual regurgitation by: obtaining, for each of a plurality of example cases, an example position of an implant device implanted in a heart and a corresponding example imaging region position; determining, based on the example position of the implant device for each example case and the identified position of the implant device, a most similar example case; and defining the at least one imaging region for assessing residual regurgitation based on the example imaging region position for the most similar example case.

In some examples, the processing system is further configured to control a display device to output an indication of each defined imaging region.

This allows a user to assess a suitability of the defined imaging region(s) for assessing residual regurgitation.

In some examples, the processing system is configured to control an ultrasound imaging device to acquire ultrasound imaging data of each defined imaging region for assessing residual regurgitation.

In some examples, the processing system is configured to control the ultrasound imaging device to acquire the ultrasound imaging data in a 3D color Doppler mode.

3D Doppler imaging data of the region covers the entire region (i.e. the entire 3D volume of the region), improving a likelihood that the ultrasound imaging data captures a location of residual regurgitation.

In some examples, the processing system is configured to control the ultrasound imaging device to acquire the ultrasound imaging data in an X-plane format.

X-plane imaging enables the ultrasound imaging data to be acquired at a much higher frame rate than 3D Doppler imaging.

There is also provided an ultrasound imaging system, comprising: an ultrasound imaging device; and the processing system described above.

According to examples in accordance with another aspect of the invention, there is provided a computer-implemented method for defining at least one imaging region for assessing residual regurgitation, the method comprising: obtaining medical imaging data of a heart of a subject, the medical imaging data capturing an implant device implanted in the heart; processing the medical imaging data to identify a position of the implant device; determining one or more locations of expected residual regurgitation based on at least the identified position of the implant device; and defining, based on the one or more locations of expected residual regurgitation, at least one imaging region for assessing residual regurgitation.

There is also provided a computer program product comprising computer program code means which, when executed on a computing device having a processing system, cause the processing system to perform all of the steps of the method described above.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 illustrates an exemplary ultrasound system;
Fig. 2 illustrates an ultrasound imaging system, according to an embodiment of the invention;
Fig. 3 illustrates an example of the determination of one or more locations of expected residual regurgitation based on a position and orientation of an implant device;
Fig. 4 illustrates an example of the determination of the one or more locations of expected residual regurgitation based on a position of the implant device and a position of an anatomical feature; and
Fig. 5 illustrates a computer-implemented method for defining at least one imaging region for assessing residual regurgitation, according to an embodiment of the invention.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a system and method for defining at least one imaging region for assessing residual regurgitation. Medical imaging data of a heart having an implant device is obtained and processed to identify at least a position of the implant device. One or more locations of expected residual regurgitation are determined based at least one the identified position of the implant device and are used to define at least one imaging region for assessing residual regurgitation.

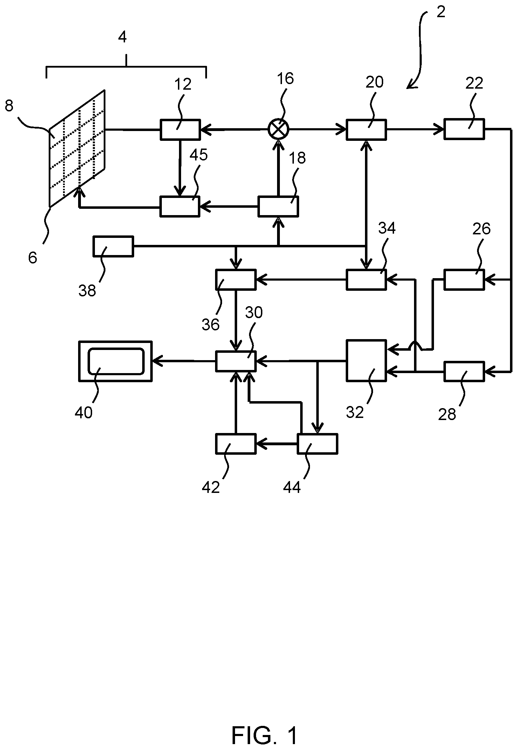

The general operation of an exemplary ultrasound system will first be described, with reference to Fig. 1, and with emphasis on the signal processing function of the system since this invention relates to the processing of the signals measured by the transducer array.

The system comprises an array transducer probe 4 which has a transducer array 6 for transmitting ultrasound waves and receiving echo information. The transducer array 6 may comprise CMUT transducers; piezoelectric transducers, formed of materials such as PZT or PVDF; or any other suitable transducer technology. In this example, the transducer array 6 is a two-dimensional array of transducers 8 capable of scanning either a 2D plane or a three dimensional volume of a region of interest. In another example, the transducer array may be a 1D array.

The transducer array 6 is coupled to a microbeamformer 12 which controls reception of signals by the transducer elements. Microbeamformers are capable of at least partial beamforming of the signals received by sub-arrays, generally referred to as "groups" or "patches", of transducers as described in US Patents 5,997,479 (Savord et al.), 6,013,032 (Savord), and 6,623,432 (Powers et al.).

It should be noted that the microbeamformer is entirely optional. Further, the system includes a transmit/receive (T/R) switch 16, which the microbeamformer 12 can be coupled to and which switches the array between transmission and reception modes, and protects the main beamformer 20 from high energy transmit signals in the case where a microbeamformer is not used and the transducer array is operated directly by the main system beamformer. The transmission of ultrasound beams from the transducer array 6 is directed by a transducer controller 18 coupled to the microbeamformer by the T/R switch 16 and a main transmission beamformer (not shown), which can receive input from the user's operation of the user interface or control panel 38. The controller 18 can include transmission circuitry arranged to drive the transducer elements of the array 6 (either directly or via a microbeamformer) during the transmission mode.

In a typical line-by-line imaging sequence, the beamforming system within the probe may operate as follows. During transmission, the beamformer (which may be the microbeamformer or the main system beamformer depending upon the implementation) activates the transducer array, or a sub-aperture of the transducer array. The sub-aperture may be a one dimensional line of transducers or a two dimensional patch of transducers within the larger array. In transmit mode, the focusing and steering of the ultrasound beam generated by the array, or a sub-aperture of the array, are controlled as described below.

Upon receiving the backscattered echo signals from the subject, the received signals undergo receive beamforming (as described below), in order to align the received signals, and, in the case where a sub-aperture is being used, the sub-aperture is then shifted, for example by one transducer element. The shifted sub-aperture is then activated and the process repeated until all of the transducer elements of the transducer array have been activated.

For each line (or sub-aperture), the total received signal, used to form an associated line of the final ultrasound image, will be a sum of the voltage signals measured by the transducer elements of the given sub-aperture during the receive period. The resulting line signals, following the beamforming process below, are typically referred to as radio frequency (RF) data. Each line signal (RF data set) generated by the various sub-apertures then undergoes additional processing to generate the lines of the final ultrasound image. The change in amplitude of the line signal with time will contribute to the change in brightness of the ultrasound image with depth, wherein a high amplitude peak will correspond to a bright pixel (or collection of pixels) in the final image. A peak appearing near the beginning of the line signal will represent an echo from a shallow structure, whereas peaks appearing progressively later in the line signal will represent echoes from structures at increasing depths within the subject.

One of the functions controlled by the transducer controller 18 is the direction in which beams are steered and focused. Beams may be steered straight ahead from (orthogonal to) the transducer array, or at different angles for a wider field of view. The steering and focusing of the transmit beam may be controlled as a function of transducer element actuation time.

Two methods can be distinguished in general ultrasound data acquisition: plane wave imaging and "beam steered" imaging. The two methods are distinguished by a presence of the beamforming in the transmission ("beam steered" imaging) and/or reception modes (plane wave imaging and "beam steered" imaging).

Looking first to the focusing function, by activating all of the transducer elements at the same time, the transducer array generates a plane wave that diverges as it travels through the subject. In this case, the beam of ultrasonic waves remains unfocused. By introducing a position dependent time delay to the activation of the transducers, it is possible to cause the wave front of the beam to converge at a desired point, referred to as the focal zone. The focal zone is defined as the point at which the lateral beam width is less than half the transmit beam width. In this way, the lateral resolution of the final ultrasound image is improved.

For example, if the time delay causes the transducer elements to activate in a series, beginning with the outermost elements and finishing at the central element(s) of the transducer array, a focal zone would be formed at a given distance away from the probe, in line with the central element(s). The distance of the focal zone from the probe will vary depending on the time delay between each subsequent round of transducer element activations. After the beam passes the focal zone, it will begin to diverge, forming the far field imaging region. It should be noted that for focal zones located close to the transducer array, the ultrasound beam will diverge quickly in the far field leading to beam width artifacts in the final image. Typically, the near field, located between the transducer array and the focal zone, shows little detail due to the large overlap in ultrasound beams. Thus, varying the location of the focal zone can lead to significant changes in the quality of the final image.

It should be noted that, in transmit mode, only one focus may be defined unless the ultrasound image is divided into multiple focal zones (each of which may have a different transmit focus).

In addition, upon receiving the echo signals from within the subject, it is possible to perform the inverse of the above described process in order to perform receive focusing. In other words, the incoming signals may be received by the transducer elements and subject to an electronic time delay before being passed into the system for signal processing. The simplest example of this is referred to as delay-and-sum beamforming. It is possible to dynamically adjust the receive focusing of the transducer array as a function of time.

Looking now to the function of beam steering, through the correct application of time delays to the transducer elements it is possible to impart a desired angle on the ultrasound beam as it leaves the transducer array. For example, by activating a transducer on a first side of the transducer array followed by the remaining transducers in a sequence ending at the opposite side of the array, the wave front of the beam will be angled toward the second side. The size of the steering angle relative to the normal of the transducer array is dependent on the size of the time delay between subsequent transducer element activations.

Further, it is possible to focus a steered beam, wherein the total time delay applied to each transducer element is a sum of both the focusing and steering time delays. In this case, the transducer array is referred to as a phased array.

In case of the CMUT transducers, which require a DC bias voltage for their activation, the transducer controller 18 can be coupled to control a DC bias control 45 for the transducer array. The DC bias control 45 sets DC bias voltage(s) that are applied to the CMUT transducer elements.

For each transducer element of the transducer array, analog ultrasound signals, typically referred to as channel data, enter the system by way of the reception channel. In the reception channel, partially beamformed signals are produced from the channel data by the microbeamformer 12 and are then passed to a main receive beamformer 20 where the partially beamformed signals from individual patches of transducers are combined into a fully beamformed signal, referred to as radio frequency (RF) data. The beamforming performed at each stage may be carried out as described above, or may include additional functions. For example, the main beamformer 20 may have 128 channels, each of which receives a partially beamformed signal from a patch of dozens or hundreds of transducer elements. In this way, the signals received by thousands of transducers of a transducer array can contribute efficiently to a single beamformed signal.

The beamformed reception signals are coupled to a signal processor 22. The signal processor 22 can process the received echo signals in various ways, such as: band-pass filtering; decimation; I and Q component separation; and harmonic signal separation, which acts to separate linear and nonlinear signals so as to enable the identification of nonlinear (higher harmonics of the fundamental frequency) echo signals returned from tissue and micro-bubbles. The signal processor may also perform additional signal enhancement such as speckle reduction, signal compounding, and noise elimination. The band-pass filter in the signal processor can be a tracking filter, with its pass band sliding from a higher frequency band to a lower frequency band as echo signals are received from increasing depths, thereby rejecting noise at higher frequencies from greater depths that is typically devoid of anatomical information.

The beamformers for transmission and for reception are implemented in different hardware and can have different functions. Of course, the receiver beamformer is designed to take into account the characteristics of the transmission beamformer. In Fig. 1 only the receiver beamformers 12, 20 are shown, for simplicity. In the complete system, there will also be a transmission chain with a transmission micro beamformer, and a main transmission beamformer.

The function of the micro beamformer 12 is to provide an initial combination of signals in order to decrease the number of analog signal paths. This is typically performed in the analog domain.

The final beamforming is done in the main beamformer 20 and is typically after digitization.

The transmission and reception channels use the same transducer array 6 which has a fixed frequency band. However, the bandwidth that the transmission pulses occupy can vary depending on the transmission beamforming used. The reception channel can capture the whole transducer bandwidth (which is the classic approach) or, by using bandpass processing, it can extract only the bandwidth that contains the desired information (e.g. the harmonics of the main harmonic).

The RF signals may then be coupled to a B mode (i.e. brightness mode, or 2D imaging mode) processor 26 and a Doppler processor 28. The B mode processor 26 performs amplitude detection on the received ultrasound signal for the imaging of structures in the body, such as organ tissue and blood vessels. In the case of line-by-line imaging, each line (beam) is represented by an associated RF signal, the amplitude of which is used to generate a brightness value to be assigned to a pixel in the B mode image. The exact location of the pixel within the image is determined by the location of the associated amplitude measurement along the RF signal and the line (beam) number of the RF signal. B mode images of such structures may be formed in the harmonic or fundamental image mode, or a combination of both as described in US Pat. 6,283,919 (Roundhill et al.) and US Pat. 6,458,083 (Jago et al.) The Doppler processor 28 processes temporally distinct signals arising from tissue movement and blood flow for the detection of moving substances, such as the flow of blood cells in the image field. The Doppler processor 28 typically includes a wall filter with parameters set to pass or reject echoes returned from selected types of materials in the body.

The structural and motion signals produced by the B mode and Doppler processors are coupled to a scan converter 32 and a multi-planar reformatter 44. The scan converter 32 arranges the echo signals in the spatial relationship from which they were received in a desired image format. In other words, the scan converter acts to convert the RF data from a cylindrical coordinate system to a Cartesian coordinate system appropriate for displaying an ultrasound image on an image display 40. In the case of B mode imaging, the brightness of pixel at a given coordinate is proportional to the amplitude of the RF signal received from that location. For instance, the scan converter may arrange the echo signal into a two dimensional (2D) sector-shaped format, or a pyramidal three dimensional (3D) image. The scan converter can overlay a B mode structural image with colors corresponding to motion at points in the image field, where the Doppler-estimated velocities to produce a given color. The combined B mode structural image and color Doppler image depicts the motion of tissue and blood flow within the structural image field. The multi-planar reformatter will convert echoes that are received from points in a common plane in a volumetric region of the body into an ultrasound image of that plane, as described in US Pat. 6,443,896 (Detmer). A volume renderer 42 converts the echo signals of a 3D data set into a projected 3D image as viewed from a given reference point as described in US Pat. 6,530,885 (Entrekin et al.).

The 2D or 3D images are coupled from the scan converter 32, multi-planar reformatter 44, and volume renderer 42 to an image processor 30 for further enhancement, buffering and temporary storage for display on an image display 40. The imaging processor may be adapted to remove certain imaging artifacts from the final ultrasound image, such as: acoustic shadowing, for example caused by a strong attenuator or refraction; posterior enhancement, for example caused by a weak attenuator; reverberation artifacts, for example where highly reflective tissue interfaces are located in close proximity; and so on. In addition, the image processor may be adapted to handle certain speckle reduction functions, in order to improve the contrast of the final ultrasound image.

In addition to being used for imaging, the blood flow values produced by the Doppler processor 28 and tissue structure information produced by the B mode processor 26 are coupled to a quantification processor 34. The quantification processor produces measures of different flow conditions such as the volume rate of blood flow in addition to structural measurements such as the sizes of organs and gestational age. The quantification processor may receive input from the user control panel 38, such as the point in the anatomy of an image where a measurement is to be made.

Output data from the quantification processor is coupled to a graphics processor 36 for the reproduction of measurement graphics and values with the image on the display 40, and for audio output from the display device 40. The graphics processor 36 can also generate graphic overlays for display with the ultrasound images. These graphic overlays can contain standard identifying information such as patient name, date and time of the image, imaging parameters, and the like. For these purposes the graphics processor receives input from the user interface 38, such as patient name. The user interface is also coupled to the transmit controller 18 to control the generation of ultrasound signals from the transducer array 6 and hence the images produced by the transducer array and the ultrasound system. The transmit control function of the controller 18 is only one of the functions performed. The controller 18 also takes account of the mode of operation (given by the user) and the corresponding required transmitter configuration and band-pass configuration in the receiver analog to digital converter. The controller 18 can be a state machine with fixed states.

The user interface is also coupled to the multi-planar reformatter 44 for selection and control of the planes of multiple multi-planar reformatted (MPR) images which may be used to perform quantified measures in the image field of the MPR images.

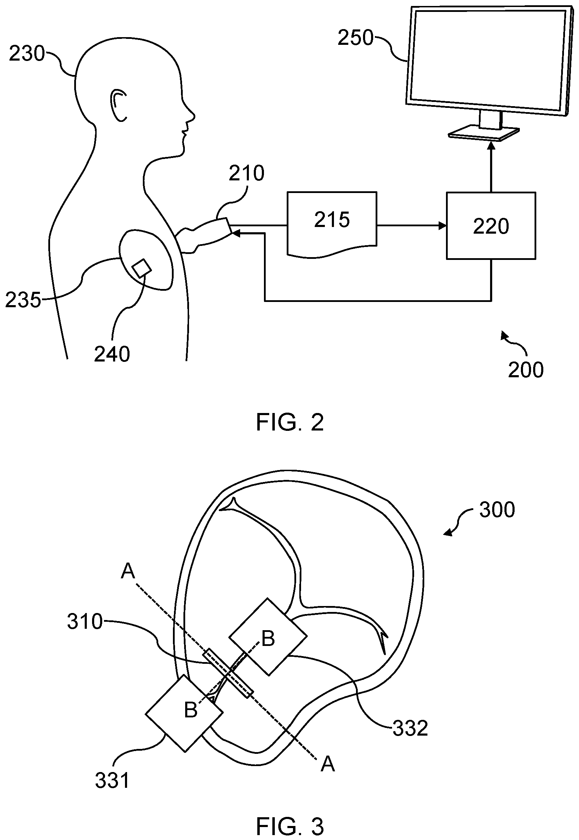

Fig. 2 illustrates an ultrasound imaging system 200, according to an embodiment of the invention. The ultrasound imaging system 200 comprises an ultrasound imaging device 210 and a processing system 220. The processing system 220 is, itself, an embodiment of the invention.

The ultrasound imaging device 210 may be any ultrasound imaging device capable of acquiring ultrasound imaging data suitable for assessing residual regurgitation. For instance, the ultrasound imaging device may be capable of acquiring ultrasound imaging data in a 3D color Doppler mode and/or of acquiring X-plane ultrasound imaging data.

The processing system 220 is configured to obtain medical imaging data 215 of a heart 235 of a subject 230. The subject 230 has an implant device 240 implanted in the heart, and the medical imaging data 215 captures the implant device. The implant device is a device implanted in a valve of the heart to treat regurgitation. For instance, the implant device may be implanted in the mitral valve to treat mitral regurgitation, or in the tricuspid valve to treat tricuspid regurgitation. In some examples, the medical imaging data may be acquired during the procedure in which the implant device was implanted. This allows residual regurgitation to be assessed immediately after fitting the implant device; in this way, a further implant device may be fitted, if the assessment of residual regurgitation indicates this is needed, during the same procedure as the original implant device.

In Fig. 2, the medical imaging data 215 is acquired by the ultrasound imaging device 210; however, in some examples, the medical imaging data may be acquired by another ultrasound imaging device or by another type of imaging device. The medical imaging data may be any medical imaging device capable of acquiring imaging data of a subject's heart in which an implant device implanted in the heart can be detected, and from which a position of the implant device in 3D space may be determined; for example, the medical imaging data may be 3D medical imaging data (e.g. 3D ultrasound imaging data), or the medical imaging data may be 2D medical imaging data comprising a projection of a 3D volume from which the position of an object in the 2D imaging data may be reconstructed in 3D space (e.g. using 2D-3D registration). For instance, the medical imaging data may be 3D transesophageal echocardiography (TEE) imaging data, 3D transthoracic echocardiography (TTE) imaging data, 3D intracardiac echocardiography imaging data, or fluoroscopy imaging data. In examples in which the medical imaging data is 3D ultrasound imaging data, the 3D ultrasound imaging data may be B-mode imaging data or Doppler imaging data. In some examples, the medical imaging data may comprise more than one type of imaging data; for instance, the medical imaging data may comprise both 3D ultrasound imaging data and fluoroscopy imaging data.

In examples in which the medical imaging data 215 is acquired by a device other than the ultrasound imaging device 210, the position at which the medical imaging data is acquired relative to the position of the ultrasound imaging device 210 is known. For instance, the imaging device used to acquire the medical imaging data 215 may be co-registered with the ultrasound imaging device 210.

In Fig. 2, the processing system 220 obtains the medical imaging data 215 directly from the ultrasound imaging device 210; however, in some examples, the medical imaging data may be obtained from a memory unit (not shown in Fig. 2) storing the medical imaging data.

Having obtained the medical imaging data 215, the processing system 220 is configured to process the medical imaging data to identify a position of the implant device 240. In examples in which the medical imaging data comprises more than one type of imaging data, each type of imaging data in the medical imaging data may be used to identify the position of the implant device (for instance, a combination of 3D ultrasound imaging data and fluoroscopy data may be used to identify the position of the implant device).

The processing system 220 may be configured to identify the position of the implant device 240 automatically. For example, the processing system may be configured to identify the position of the implant device by processing the medical imaging data 215 using a first segmentation algorithm. Any suitable segmentation algorithm (such as a model-based segmentation algorithm or a machine-learning segmentation algorithm). In another example, the processing system may be configured to identify the position of the implant device using template matching (i.e. by obtaining a template of the implant device and identifying a region in the medical imaging data that matches the template), or using an object detection algorithm.

Alternatively, the processing system 220 may be configured to identify the position of the implant device by processing the medical imaging data 215 to generate an image of the heart, controlling a display device to display the image, and receiving a user input indicating a position of the implant device within the image.

The processing system 220 is then configured to determine one or more locations of expected residual regurgitation based on at least the identified position of the implant device 240.

In some examples, the one or more locations of expected residual regurgitation may be determined based only on the identified position of the implant device 240. For instance, if the implant device has been implanted in a mitral valve, and the ultrasound device 210 is placed at a mid-esophageal position, the one or more locations of expected residual regurgitation may be determined between the position of the implant device and the position of the ultrasound device 210 (which is implicitly known from the medical imaging data 215: in the case of medical imaging data acquired by the ultrasound device, the position of the ultrasound device is at the apex or frustum tip of the imaging volume; in the case of medical imaging data acquired by another imaging device, the position of the ultrasound device is known by co-registration of the imaging devices).

In other examples, the one or more locations of expected residual regurgitation may be determined based on the identified position of the implant device 240 in combination with further information. The further information may, for example, comprise one or more of: an orientation of the implant device, a position of one or more anatomical features, pathology information for the subject, and/or an example position of an implant device and corresponding example imaging region for each of a plurality of example cases. The use of further information in combination with the position of the implant device enables a more precise determination of one or more locations of expected residual regurgitation (resulting in a smaller imaging region for assessing residual regurgitation and/or fewer imaging regions for assessing residual regurgitation).

In some examples, the determination of the one or more locations of expected residual regurgitation may be further based on the orientation of the implant device 240. In other words, the processing system 220 may be configured to process the medical imaging data 215 to identify an orientation of the implant device, and to determine the one or more location of expected residual regurgitation based on at least the identified position and orientation of the implant device 240. The orientation of the implant device may be identified based on the shape of the implant device and/or based on the placement of the imaging device used to acquire the medical imaging data.

The position and orientation of the implant device may be used to define an axis along which residual regurgitation is likely to occur. This is based on the recognition that an implant device for treating regurgitation is implanted in the relevant valve of the heart such that a perpendicular axis of the implant device (i.e. an axis perpendicular to the long axis and the up/down axis of the implant device) is approximately parallel to the coaptation line between the leaflets of the valve that are pulled together by the implant device. Residual regurgitation is likely to occur along the perpendicular axis on one side of the implant device (but with only the position and orientation of the implant device, and no anatomical context, it is not possible to predict the side of the implant device on which residual regurgitation will occur). Thus, the processing system 220 may be configured to determine, as locations of expected residual regurgitation, a first location that is a predetermined distance from the implant device along the perpendicular axis on a first side of the implant device, and a second location that is the predetermined distance from the implant device along the perpendicular axis on a second, opposing side of the implant device.

Fig. 3 illustrates an example of the determination of the one or more locations of expected residual regurgitation based on a position and orientation of the implant device. Fig. 3 shows a tricuspid valve 300 of a heart, with an implant device 310 pulling together leaflets in the tricuspid valve. A long axis A-A of the implant device connects the leaflets pulled together by the implant device. The up/down axis of the implant device is perpendicular to the page.

By identifying a position and orientation of the implant device 310, the long axis A-A, the up/down axis and the perpendicular axis B-B, which is perpendicular to the long axis and up/down axis, may be identified. The perpendicular axis B-B is close to parallel to the coaptation line between the leaflets that are pulled together by the implant device. A first location 331 of expected residual regurgitation is identified along the perpendicular axis B-B on a first side of the implant device, and a second location 332 of expected residual regurgitation is identified along the perpendicular axis B-B on a second side of the implant device.

Returning to Fig. 2, in some examples where the medical imaging data is 3D ultrasound imaging data, the determination of the one or more locations of expected residual regurgitation may be further based on a position of one or more anatomical features. In other words, the processing system 220 may be configured to process the medical imaging data 215 to identify a position of one or more anatomical features, and to determine the one or more location of expected residual regurgitation based on at least the identified position of the implant device 240 and the one or more anatomical features. In this way, the position of the implant device 240 within the valve can be determined and used to determine the one or more location of expected residual regurgitation.

The one or more anatomical features may be any anatomical features suitable for determining the position of the implant device 240 with respect to the valve of the heart in which the implant device is implanted. For instance, the one or more anatomical features may include one or more of: an annulus of the valve in which the implant device is implanted; the heart chambers adjacent to the valve in which the implant device is implanted (i.e. the left atrium and left ventricle if the implant device is implanted in a mitral valve, and the right atrium and right ventricle if the implant device is implanted in a tricuspid valve); a connection point (commissure) between adjacent leaflets on the annulus of the valve in which the implant device is implanted; and/or a line of coaptation between adjacent leaflets on the annulus of the valve in which the implant device is implanted. Other suitable anatomical features will be apparent to the skilled person.

The processing system 220 may, for example, be configured to identify the position of the one or more anatomical features by processing the medical imaging data using a second segmentation algorithm. Any suitable segmentation algorithm may be used to identify the position of the one or more anatomical features. For instance, the second segmentation algorithm may be a model-based segmentation algorithm or a machine-learning segmentation algorithm.

As previously stated, in some examples, the position of the implant device may be identified using a first segmentation algorithm. In examples in which segmentation is used to identify both the position of the implant device and the position of one or more anatomical features, a single segmentation algorithm may be used to identify both the position of the implant device and the position of the one or more anatomical features (i.e. the first segmentation algorithm and the second segmentation algorithm may be the same algorithm, e.g. a machine-learning algorithm). Alternatively, the second segmentation algorithm used to identify the position of the one or more anatomical features may be a different segmentation algorithm to the first segmentation algorithm used to identify the position of the implant device.

In other examples, the processing system 220 may be configured to identify the position of the one or more anatomical features by processing the medical imaging data 215 using a landmark detection algorithm.

In yet other examples, the processing system 220 may be configured to identify the position of the one or more anatomical features by processing the medical imaging data 215 to generate an image of the heart, controlling a display device to display the image, and receiving a user input indicating a position of the one or more anatomical features within the image.

In yet other examples, the processing system 220 may be configured to identify the position of the one or more anatomical features by transmitting the medical imaging data 215 to another processing system, which is configured to identify the position of the one or more anatomical features (e.g. using any of the methods described above). The processing system 220 may then receive an identification of the position of the one or more anatomical features from the other processing system.

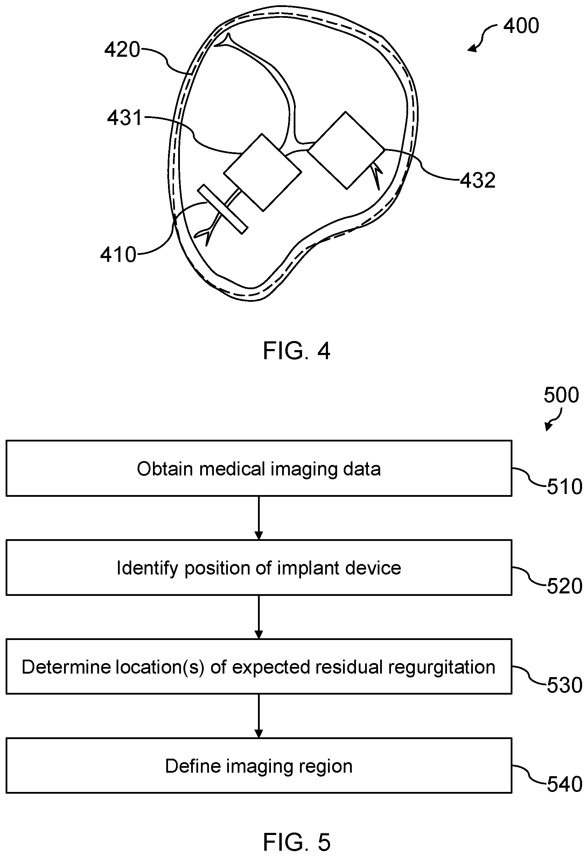

Fig. 4 illustrates an example of the determination of the one or more locations of expected residual regurgitation based on a position of the implant device and a position of an anatomical feature. Fig. 4 shows a tricuspid valve 400 of a heart, with an implant device 410 pulling together leaflets in the tricuspid valve. The annulus of the tricuspid valve has been segmented; the dotted line 420 indicates the position of the detected annulus.

The identified position of the implant device 410 and the identified position of the annulus 420 together show that the implant device is located at the anterior-septal coaptation line, close to the annulus. As the implant device is close to the annulus, residual regurgitation may be expected more centrally in the anterior-septal coaptation area. Thus, a first location 431 of expected residual regurgitation is identified in the anterior-septal coaptation area, close to the center of the tricuspid valve. A second location 432 of expected residual regurgitation is identified at the posterior-septal coaptation line (as clinical evidence has shown that this is the second most likely location for residual regurgitation when an implant device is located at the anterior-septal coaptation line). In some examples, a third location of expected residual regurgitation (not shown in Fig. 4) may be identified at the anterior-posterior commissure.

Returning to Fig. 2, in some examples, the determination of the one or more locations of expected residual regurgitation may be further based on pathology information. In other words, the processing system 220 may be configured to obtain pathology information for the subject, and to determine the one or more location of expected residual regurgitation based on at least the identified position of the implant device 240 and the pathology information.

The pathology information may be any information relating to the condition for which the implant device was implanted in the valve of the heart (e.g. mitral regurgitation or tricuspid regurgitation). The pathology information may, for example, be obtained from an electronic medical record for the subject (i.e. obtained by accessing a clinical database containing the electronic medical record) or by receiving a user input.

For instance, the pathology information may comprise an indication of whether the regurgitation is primary (i.e. caused by a structural defect, such as rupture of chordae holding the leaflets) or secondary (e.g. caused by a deformation such as dilation of the annulus, which makes the valve area larger, so that even a structurally intact valve can no longer cover the entire orifice). If the regurgitation is primary, the pathology information may comprise an indication of the segment or subsegment of the valve that is affected. If the pathology information indicates that the regurgitation is primary, the one or more locations of expected residual regurgitation may be identified close to the implant device. If the pathology information indicates that the regurgitation is secondary, residual regurgitation may also be expected further from the implant device.

In some examples, a combination of the position of the implant device, the position of one or more anatomical features and pathology information may be used to determine the one or more locations of expected residual regurgitation. For instance, if the pathology information indicates a prolapse in two segments of a leaflet, and the position of the implant device and the one or more anatomical features indicates that the implant device is located in one of these segments, the other segment may be identified as a location of expected residual regurgitation.

In examples in which the processing system 220 has identified the position of one or more anatomical features, the processing system may be configured to obtain the pathology information by processing the identified position of the one or more anatomical features. For instance, a detailed segmentation of leaflets of the valve in which the implant device is located would enable the identification of any areas in which the leaflets do not close. Residual regurgitation is more likely to occur in these areas, so any area in which the leaflets do not close may be identified as a location of expected residual regurgitation.

Returning to Fig. 2, having determined one or more locations of expected residual regurgitation, the processing system 220 is configured to define, based on the one or more locations of expected residual regurgitation, at least one imaging region for assessing residual regurgitation. Defining at least one imaging region for assessing residual regurgitation comprises, for each imaging region, defining a position and size of the imaging region such that the imaging region contains a location of expected residual regurgitation, where the position of the imaging region is a position with respect to the ultrasound imaging device 210. The imaging region may be an imaging region for 3D Doppler imaging or X-plane imaging, as described below.

If the medical imaging data 215 used to determine the location(s) of expected residual regurgitation is acquired by the ultrasound imaging device 210, the imaging region will be defined with respect to the ultrasound imaging device as a matter of course. If a separate device (co-registered with the ultrasound imaging device 210) is used to acquire the medical imaging data 215, a translation to the coordinates of the ultrasound imaging device 210 must be performed. The translation may be performed on the medical imaging data 215, the identified position of the imaging device 240, or the determined location(s) of expected residual regurgitation.

In some examples, where more than one location of expected residual regurgitation is determined, the processing system 220 may be configured to determine an imaging region for each determined location of expected residual regurgitation. This may particularly be the case if the locations of expected residual regurgitation are determined based only on the position of the implant device 240, or only on the position and orientation of the implant device, as it is not possible to determine at which of the locations residual regurgitation is most likely to occur based only on information about the implant device (i.e. without anatomical context or pathology information), although the processing system may, in some examples, define an imaging region for each determined location of expected residual regurgitation even where information enabling a most likely location to be identified is available.

In other examples where more than one location of expected residual regurgitation is determined, the processing system 220 may be configured to select, from the determined locations of expected residual regurgitation, a most likely location of residual regurgitation (e.g. based on the identified one or more anatomical features, where available), and to define an imaging region based on the most likely location. For instance, in the example of Fig. 4, the first location 431 is the most likely location of residual regurgitation; an imaging region may therefore be defined such that the imaging region covers the first location 431. Similarly, pathology information (alone or in combination with the identified one or more anatomical features) may be used to identify a most likely location.

In some examples, the processing system 220 may be further configured to control a display device 250 to output an indication of each defined imaging region. For instance, the processing system 220 may process the medical imaging data 215 to generate an image of the heart, overlay an indication of each defined imaging region on the generated image, and control the display device to output the generated image with the overlay. The processing system may then be configured to receive a user input indicating which of a plurality of imaging regions should be used to assess residual regurgitation, and/or indicating an adjustment to the position and/or size of an imaging region. For instance, where the processing system determines locations of expected residual regurgitation based only on information about the implant device 240, and determines an imaging region for each location, a clinically-trained user would be able to identify which location is more suitable based on the anatomical context of the image (for example, in the example of Fig. 3, a user would select the second location 332 as the location at which residual regurgitation is most likely to occur). In another example, a user with clinical expertise may determine that the defined imaging region would capture only part of the regurgitant jet, and provide a user input repositioning and/or resizing the imaging region.

In some examples, the processing system 220 may be configured to define the at least one imaging region for assessing residual regurgitation by obtaining an example position of an implant device implanted in a heart and a corresponding example imaging region position for each of a plurality of example cases. The plurality of example cases may, for example, be obtained from a database storing the example cases. The processing system may then be configured to determine a most similar example case to the present case, and to define the at least one imaging region based on the example imaging region position for the most similar example case.

In each of the plurality of example cases, the example imaging region is an imaging region that was used to assess residual regurgitation in the example case. The example imaging region may have been determined by a clinician or by performing any of the techniques described above. For instance, the processing system 220 may, each time the processing system defines an imaging region for assessing residual regurgitation, store, in a database, the defined imaging region and information used to define the imaging region (e.g. the medical imaging data, the position of the implant device, and, if used, the orientation of the device, the position of one or more anatomical features, and/or pathology information). In examples in which more than one imaging region is defined, residual regurgitation may be assessed at each imaging region, and only an imaging region at which residual regurgitation is found (or the imaging region at which residual regurgitation is greatest) may be stored as the example imaging region.

The processing system 220 may be configured to determine a most similar example case based at least on the example position of the implant device for each example case and the identified position of the implant device in the present case. Where additional information is available (e.g. an identified orientation of the implant device, one or more identified anatomical features, and/or pathology information), this information may also be used to determine the most similar case.

The processing system 220 may be configured to define the at least one imaging region based on the example imaging region position for the most similar example case by, for example, selecting one of a plurality of determined locations of expected residual regurgitation based on the example imaging region (e.g. selecting the determined location that has a most similar position to the example imaging region), and defining an imaging region that contains the selected location. In another example, the processing system may be configured to define the at least one imaging region based on the example imaging region position for the most similar example case by using the example imaging region to determine the one or more locations of expected residual regurgitation.

In some examples, having defined at least one imaging region, the processing system 220 may be further configured to control the ultrasound imaging device 210 to acquire ultrasound imaging data of each defined imaging region for assessing residual regurgitation. The ultrasound imaging data may be any type of ultrasound imaging data that allows residual regurgitation to be assessed. For instance, the processing system may be configured to control the ultrasound imaging device to acquire the ultrasound imaging data in a 3D color Doppler mode. Alternatively, processing system may be configured to control the ultrasound imaging device to acquire the ultrasound imaging data in an X-plane format, where at least one plane is a Doppler plane (for instance, both planes may be Doppler planes, or one plane may be a Doppler plane and the other plane may be a B-mode plane).

In some examples, the processing system 220 may be further configured to receive the ultrasound imaging data acquired by the ultrasound imaging device. The processing system may be configured to process the ultrasound imaging data to determine a measure of residual regurgitation for each defined imaging region.

In some examples, the processing system 220 may be additionally or alternatively configured to process the ultrasound imaging data to generate an image, and to control the display device 250 to display the generated image. In examples in which the processing system is configured to determine a measure of residual regurgitation for each defined imaging region, the processing system may be configured to control the display device to display the determined measure of residual regurgitation for the imaging region corresponding to the generated image. In examples in which more than one imaging region is defined, a measure of residual regurgitation may be determined for each imaging region, and an image may be generated and displayed only for an imaging region for which residual regurgitation is detected (i.e. for which the measure of residual regurgitation exceeds a predetermined threshold), or only the imaging region for which residual regurgitation is greatest.

Fig. 5 illustrates a computer-implemented method 500 for defining at least one imaging region for assessing residual regurgitation, according to an embodiment of the invention.

The method 500 starts at step 510, at which medical imaging data of a heart of a subject is obtained. The medical imaging data captures an implant device implanted in the heart.

At step 520, the medical imaging data is processed to identify a position of the implant device.

At step 530, one or more locations of expected residual regurgitation are determined based on at least the identified position of the implant device. The one or more locations of expected residual regurgitation may be determined using any of the techniques described above.

At step 540, at least one imaging region for assessing residual regurgitation is defined based on the one or more locations of expected residual regurgitation.

In some examples, the method may further comprise a step (not shown in Fig. 5) of controlling an ultrasound imaging device to acquire ultrasound imaging data of each defined imaging region for assessing residual regurgitation.

In some examples, the method may further comprise the steps (not shown in Fig. 5) of receiving the ultrasound imaging data of each defined imaging region and processing the ultrasound imaging data of each defined imaging region to determine a measure of residual regurgitation for each imaging region.

In some examples, the method may further comprise a steps (not shown in Fig. 5) of processing the ultrasound imaging data to generate an image and controlling a display device to display the generated image. In some examples, the image may be generated only for an imaging region for which residual regurgitation is detected, or only the imaging region for which residual regurgitation is greatest.

It will be understood that the disclosed methods are computer-implemented methods. As such, there is also proposed a concept of a computer program comprising code means for implementing any described method when said program is run on a processing system.

The skilled person would be readily capable of developing a processing system for carrying out any herein described method. Thus, each step of a flow chart may represent a different action performed by a processing system, and may be performed by a respective module of the processing system.

As discussed above, the system makes use of a processing system to perform the data processing. The processing system can be implemented in numerous ways, with software and/or hardware, to perform the various functions required. The processing system typically employs one or more microprocessors that may be programmed using software (e.g. microcode) to perform the required functions. The processing system may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processing system may be associated with one or more storage media such as volatile and non-volatile computer memory such as RAM, PROM, EPROM, and EEPROM. The storage media may be encoded with one or more programs that, when executed on one or more processing systems and/or controllers, perform the required functions. Various storage media may be fixed within a processing system or controller may be transportable, such that the one or more programs stored thereon can be loaded into a processing system.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Functions implemented by a processing system may be implemented by a single processing system or by multiple separate processing units which may together be considered to constitute a "processor". Such processing units may in some cases be remote from each other and communicate with each other in a wired or wireless manner.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

## Claims

1. A processing system (220) for defining at least one imaging region for assessing residual regurgitation, the processing system being configured to:
obtain medical imaging data (215) of a heart (235) of a subject (230), the medical imaging data capturing an implant device (240, 310, 410)) implanted in the heart;
process the medical imaging data to identify a position of the implant device;
determine one or more locations (331, 332, 431, 432) of expected residual regurgitation based on at least the identified position of the implant device; and
define, based on the one or more locations of expected residual regurgitation, at least one imaging region for assessing residual regurgitation.

2. The processing system (220) of claim 1, wherein:
the processing system is further configured to process the medical imaging data (215) to identify an orientation of the implant device (240, 310, 410); and
the determination of the one or more locations (331, 332, 431, 432) of expected residual regurgitation is further based on the identified orientation of the implant device.

3. The processing system (220) of claim 1 or 2, wherein the processing system is configured to identify the position of the implant device (240, 310, 410) by processing the medical imaging data (215) using a first segmentation algorithm.

4. The processing system (220) of any of claims 1 to 3, wherein the medical imaging data (215) is 3D ultrasound medical imaging data.

5. The processing system (220) of claim 4, wherein:
the processing system is further configured to process the medical imaging data (215) to identify a position of one or more anatomical features; and
the determination of the one or more locations (331, 332, 431, 432) of expected residual regurgitation is further based on the identified position of the one or more anatomical features.

6. The processing system (220) of claim 5, wherein the processing system is configured to identify the position of the one or more anatomical features by processing the medical imaging data (215) using a second segmentation algorithm.

7. The processing system (220) of any of claims 1 to 6, wherein:
the processing system is further configured to obtain pathology information for the subject; and
the determination of the one or more locations (331, 332, 431, 432) of expected residual regurgitation is further based on the pathology information.

8. The processing system (220) of any of claims 1 to 7, wherein the processing system is configured to define the at least one imaging region for assessing residual regurgitation by:
obtaining, for each of a plurality of example cases, an example position of an implant device implanted in a heart and a corresponding example imaging region position;
determining, based on the example position of the implant device for each example case and the identified position of the implant device (240, 310, 410), a most similar example case; and
defining the at least one imaging region for assessing residual regurgitation based on the example imaging region position for the most similar example case.

9. The processing system (220) of any of claims 1 to 8, wherein the processing system is further configured to control a display device (250) to output an indication of each defined imaging region.

10. The processing system (220) of any of claims 1 to 9, wherein the processing system is configured to control an ultrasound imaging device (210) to acquire ultrasound imaging data of each defined imaging region for assessing residual regurgitation.

11. The processing system (220) of claim 10, wherein the processing system is configured to control the ultrasound imaging device (210) to acquire the ultrasound imaging data in a 3D color Doppler mode.

12. The processing system (220) of claim 10, wherein the processing system is configured to control the ultrasound imaging device (210) to acquire the ultrasound imaging data in an X-plane format.

13. An ultrasound imaging system (200), comprising:
an ultrasound imaging device (210); and
the processing system (220) of any of claims 1 to 12.

14. A computer-implemented method (500) for defining at least one imaging region for assessing residual regurgitation, the method comprising:
obtaining medical imaging data (215) of a heart (235) of a subject (230), the medical imaging data capturing an implant device (240, 310, 410) implanted in the heart;
processing the medical imaging data to identify a position of the implant device;
determining one or more locations (331, 332, 431, 432) of expected residual regurgitation based on at least the identified position of the implant device; and
defining, based on the one or more locations of expected residual regurgitation, at least one imaging region for assessing residual regurgitation.

15. A computer program product comprising computer program code means which, when executed on a computing device having a processing system, cause the processing system to perform all of the steps of the method (500) according to claim 14.
